# EUROPEAN PATENT APPLICATION

(11) **EP 1 674 126 A1**
(43) Date of publication of application: **28.06.2006**
(21) Application number: 04771052.0
(22) Date of filing: 29.07.2004
(51) Int. Cl.: A61M 25/10

(54) **INTRA-AORTIC BALLOON CATHETER**

(30) Priority: 30.07.2003 JP 2003283202
(71) Applicant: ZEON CORPORATION, Tokyo 100-8246 (JP)
(72) Inventor: MORI, Kenji, Zeon Medical Inc., Tokyo 1050011 (JP); IIDA, Takahiro, Zeon Medical Inc., Tokyo 1050011 (JP)
(74) Representative: Vossius & Partner
(86) International application number: PCT/JP2004/010839
(87) International publication number: WO 2005/011794

(57) **Abstract**

The intra-aortic balloon catheter comprises a catheter tube 8a having a fluid flow channel 9a for balloon expansion and a blood flow channel 10a for blood pressure measurement, and a balloon 2a attached to a distal end portion of said catheter tube 8a and having an expansion/contraction portion 3a to be expanded and contracted as a result of flowing a fluid in and out through said fluid flow channel 9a. The balloon 2a is joined with said catheter tube 8a at a distal end portion 4a and proximal end portion 5a of said balloon 2a. A blood inlet 31a is formed on said catheter tube 8a, so that inside of said blood flow channel 10a communicates with outside of said catheter tube 8a. The blood inlet 31a positions on the proximal end side of a boundary 50 with said expansion/contraction portion 3a on the proximal end portion 5a of said balloon 2a.

## Description

### TECHNICAL FIELD

The present invention relates to an intra-aortic balloon catheter inserted to patient's aorta to increase a blood flow in the coronary artery, etc. by expanding and contracting the balloon, and particularly relates to an intra-aortic balloon catheter able to be suitably used by being inserted from the artery of an arm.

### BACKGROUND ART

An intra-aortic balloon catheter pumping method (hereinafter, referred to as IABP) is known as a treatment method when the cardiac functions decline. IABP is designed to assist cardiac functions by improving the blood pressure in the aorta by the insertion of a balloon catheter in the aorta and expanding and contracting the balloon portion along with the beating of the heart.

In terms of reducing pain of the patient and saving the trouble of the operator, most of catheters used for heart treatment and inspection are often inserted from the arm vessel, such as the brachial artery and radial artery, to prevent insertion from femoral artery. However, an intra-aortic balloon catheter used in IABP is hard to be formed thinner with a smaller diameter for enabling insertion from an arm vessel due to the structural constraint, so that it is inserted from the femoral artery in most cases.

However, a technique for forming a thinner intra-aortic balloon catheter has been developed in recent years (for example, refer to the Japanese Unexamined Patent Publication No. 4-343355 and the Japanese Unexamined Patent Publication No. 7-51377). Therefore, production of an intra-aortic balloon catheter formed to be thin to a degree of being able to be inserted from the brachial artery has come to be realized. Therefore, approaches of using an intra-aortic balloon catheter by inserting from the brachial artery have come to be made.

It has become clear, however, that there arises a disadvantage that changes of blood pressure in the aorta cannot be measured highly accurately with good response when inserting a conventional intra-aortic balloon catheter from the brachial artery comparing with that in the case of inserting it from the femoral artery. When accuracy of measuring blood pressure changes in the aorta is low or a time lag arises in the measurement, expansion and contraction of the balloon along with the beating of the heart become difficult and an effect of assisting the cardiac function is hard to be confirmed.

### DISCLOSURE OF THE INVENTION

The present invention was made in consideration of the above circumstances and has as an object thereof to provide an intra-aortic balloon catheter capable of measuring blood pressure changes in the aorta highly accurately with good response even when being inserted from an arm vessel, such as brachial artery.

The present inventors have been committed themselves to study for attaining the above objects, found that it is possible to obtain an intra-aortic balloon catheter capable of measuring blood pressure changes in the aorta highly accurately with good response even when being inserted from the arm vessel, such as the brachial artery, by making a blood inlet used for measuring the blood pressure in the aorta conventionally provided near a distal end of the balloon position on the proximal end side of a boundary with an expansion/contraction portion on the proximal end portion of the balloon, and completed the present invention based on the knowledge.

Namely, according to the present invention, there is provided an intra-aortic balloon catheter comprising
a catheter tube having a fluid flow channel for balloon expansion and a blood flow channel for blood pressure measurement, and
a balloon attached to a distal end portion of the catheter tube and having an expansion/contraction portion to be expanded and contracted as a result of flowing a fluid in and out through the fluid flow channel;
wherein
the balloon is joined with the catheter tube at a distal end portion and proximal end portion of the balloon,
a blood inlet is formed on the catheter tube, so that inside of the blood flow channel communicates with outside of the catheter tube, and
the blood inlet positions on the proximal end side of a boundary with the expansion/contraction portion on the proximal end portion of the balloon.

Preferably, the blood inlet positions on the proximal end side by leaving a distance of 3 to 300 mm from the boundary with the expansion/contraction portion on the proximal end portion of the balloon.

Preferably, an opening area of the blood inlet is 0.2 to 3 mm².

Preferably, the catheter tube comprises an outer tube and an inner tube, wherein at least a part of an outer surface of the inner tube is joined with an inner surface of the outer tube along with the axial direction, so that the fluid flow channel is formed inside of the outer tube, and the blood flow channel is formed in the inner tube;
a distal end of the inner tube protrudes to the distal end side from a distal end of the outer tube;
a distal end portion of the balloon is joined with the distal end portion of the inner tube, and a proximal end portion of the balloon is joined with the distal end portion of the outer tube; and
the blood inlet is formed at a joined portion of the inner tube and outer tube, and the outer tube and inner tube are joined over all circumference of an opening rim of the blood inlet.

Alternately, the catheter tube comprises an outer tube and an inner tube arranged inside of the outer tube along with the axial direction, so that the fluid flow channel is formed inside of the outer tube, and having the blood flow channel is formed inside of the inner tube;
a distal end of the inner tube protrudes to the distal end side from a distal end of the outer tube;
a distal end portion of the balloon is joined with a distal end portion of the inner tube, and a proximal end portion of the balloon is joined with a distal end portion of the outer tube;
a recess is formed on a part of the outer surface of the outer tube;
the inner tube is exposed to the outside of the outer tube at the recess; and
the blood inlet is formed on the inner tube positioning inside of the recess.

In the embodiment, preferably, the inner tube comprises a proximal side inner tube and a distal side inner tube separated from the proximal side tube;
a distal end side opening of the proximal side inner tube positions inside of the recess, and a proximal end side opening of the distal side inner tube positions inside of the recess;
the proximal end side opening and the distal end side opening face to each other by leaving a predetermined distance inside of the recess; and
the distal end side opening of the proximal side inner tube composes the blood inlet.

Note that in the embodiment, the inner tube may be composed of a single tube, and
The blood inlet may be configured by forming an opening on the inner tube wall positioning inside of the recess.

In the present invention, in another embodiment, the catheter tube comprises a two-lumen tube and a balloon supporting tube;
a first lumen composing the fluid flow channel and a second lumen composing the blood flow channel are formed in the two-lumen tube along with the axial direction;
a distal end portion of the two-lumen tube is joined with the balloon supporting tube;
a distal end portion of the balloon supporting tube is joined with a distal end portion of the balloon, and a distal end portion of the two-lumen tube is joined with a proximal end portion of the balloon; and
the blood inlet is formed on a sidewall of the two-lumen tube.

In the embodiment, preferably, a third lumen is formed inside of the balloon supporting tube along with the axial direction, and the third lumen communicates with the second lumen.

In the present invention, when the balloon is positioned in the aorta, the blood inlet positions in the blood vessel and a proximal end opening of the blood flow channel positions outside of the body.
The intra-aortic balloon catheter of the present invention is preferably used by being inserted from the arm artery.

In the intra-aortic balloon catheter of the present invention, the blood inlet used for blood pressure measurement is positioned on the proximal end side of the boundary with the expansion/contraction portion on the proximal end portion of the balloon. Therefore, even when inserting the intra-aortic balloon catheter of the present invention from the arm vessel, such as the brachial artery, blood pressure changes in the aorta can be measured highly accurately with good response.

### BRIEF DESCRIPTION OF DRAWINGS

FIG. 1 is a schematic view of an intra-aortic balloon catheter according to a first embodiment of the present invention.
FIG. 2 is a schematic view of an intra-aortic balloon catheter according to a second embodiment of the present invention.
FIG. 3 is a schematic view of an intra-aortic balloon catheter according to a third embodiment of the present invention.
FIG. 4 is a schematic view showing a state of using the intra-aortic balloon catheter shown in FIG. 1 by inserting it from the left brachial artery.
FIG. 5 is a schematic view showing a state of using an intra-aortic balloon catheter having the conventional configuration by inserting it from the left brachial artery.

### BEST MODE FOR CARRYING OUT THE INVENTION

Below, embodiments of the present invention will be explained in detail with reference to the drawings.

### First Embodiment

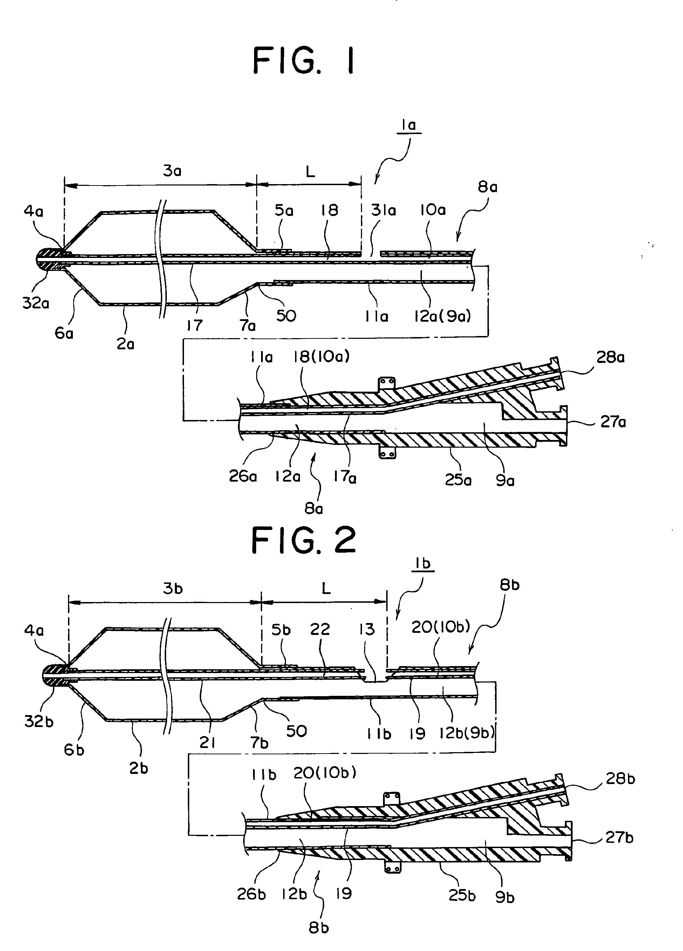

As shown in FIG. 1, an intra-aortic balloon catheter 1a according to a first embodiment of the present invention is used for IABP particularly by being inserted from the arm artery. The intra-aortic balloon catheter 1a comprises a catheter tube 8a having a fluid flow channel 9a for expanding the balloon and a blood flow channel 10a for measuring blood pressure, a balloon 2a attached at the distal end portion of the catheter tube 8a, and a tip 32a joined to the distal end portion of the balloon 2a. The catheter tube 8a comprises an outer tube 11a, inner tube 17 and a connector 25a.

The balloon 2a has a cylindrical shape and comprises an extraction/contraction portion 3a, which expands and contracts as a result of flowing in and out a fluid through the fluid flow channel 9a of the catheter 8a. A distal end portion 4a is formed on the distal end portion side of the extraction/contraction portion 3a, and a proximal end portion 5a is formed on the proximal end side of the extraction/contraction portion 3a. The distal end portion 4a and the proximal end portion 5a of the balloon 2a are used as joint margins for attaching the balloon 2a to the distal end portion of the catheter 8a and do not expand or contract by the fluid.

The extraction/contraction portion 3a of the balloon 2a is preferably provided with a distal end side taper portion 6a and a proximal end side taper portion 7a respectively having tapered shapes becoming narrower as they extend toward the distal end portion 4a and the proximal end portion 5a.

An outer diameter and length of the extraction/contraction portion 3a of the balloon 2a are determined in accordance with an inner volume of the extraction/contraction portion 3a (which affects largely on the effect of assisting cardiac functions) and an inner diameter of the artery. For example, when an inner volume of the extraction/contraction portion 3a is 20 to 50 cc, it is preferable that the outer diameter is 12 to 20 mm and the length along with the axis is 150 to 270 mm. furthermore, a film thickness of the extraction/contraction portion 3a is preferably 30 to 120 pm. Inner diameters of the distal end portion 4a and the proximal end portion 5a of the balloon 2a are preferably formed to be approximately same as the outer diameter of the catheter tube 8a, with which they are joined, respectively, and are normally in a range of 0.5 to 3.4 mm. Also, lengths of the distal end portion 4a and the proximal end portion 5a are preferably 3 to 15 mm.

A material of the balloon 2a is not particularly limited, but is preferably a material having an excellent antithrombogenicity and flex fatigue resistance, for example, composed of a synthetic resin, such as urethane based elastomer and a copolymer of polyurethane and silicone. A method of forming a balloon 2a is not particularly limited and a dipping molding method and a blow molding method are preferably used.

An outer tube 11a composing the catheter tube 8a of the intra-aortic balloon catheter 1a has a tubular shape, and a first lumen 12a is formed therein along the axial direction. The first lumen 12a is used as the fluid flow channel 9a for expanding the balloon of the catheter tube 8a. An inner diameter and thickness of the outer tube 11a are determined to enable insertion from the arm artery, such as the brachial artery, and secure a sufficient cross-sectional area of the flow channel of the fluid flow channel 9a. It is preferable that the inner diameter is 1.0 to 3.5 mm and the thickness is 0.05 to 0.3 mm, and more preferably, the inner diameter is 1.0 to 2.3 mm and the thickness is 0.05 to 0.15 mm.

Also, a length of the outer tube 11a in the axial direction is set, so that the proximal end positions outside of the body when the distal end is inserted to the aorta and is normally 400 to 800 mm. As a material of composing the outer tube 11a, for example, polyethylene, polypropyrene, polyethylene terephthalate, polyamide, polyvinyl chloride, polyurethane, fluorine resin and other synthetic resins may be used.

The inner tube 17 composing the catheter tube 8a has a tubular shape, and a second lumen 18 is formed therein along the axial direction. The second lumen 18 of the inner tube 17 is used as the blood flow channel 10a for measuring blood pressure of the catheter tube 8a. Also, the second lumen 18 can be used for leading a guide wire used at the time of inserting the intra-aortic balloon catheter 1a to the patient's aorta.

An inner diameter of the inner tube 17 is not particularly limited as far as the guide wire can be inserted, but is preferably 0.1 to 1.5 mm. A thickness of the inner tube 17 is not particularly limited as far as it can support the balloon 2a, but is preferably 0.05 to 0.4 mm. Also, a length of the inner tube 17 in the axial direction is normally 550 to 1100 mm. As a material for composing the inner tube 17, a material having a high modulus of bending and a certain degree of flexibility is preferable. Specifically, stainless, tungsten, a nickel-titan alloy and other metals, polyetherether ketone and other synthetic resins, and composite materials obtained by reinforcing a synthetic resin by metal are preferably used. Among them, in terms of having high elasticity and being hard to be deformed permanently, it is preferable to use a nickel-titan alloy exhibiting superelasticity, while in terms of being hard to be broken, it is preferable to use polyetherether ketone.

The inner tube 17 is arranged inside the first lumen 12a of the outer tube 11a and extends along with the axial direction and both ends of the inner tube 17 protrude from the outer tube 11a. At least a part of an inner surface of the outer tube 11a and an outer surface of the inner tube 17 are fixed by adhesive agent, etc.

In the present embodiment, at least at a part of the fixed portion, a blood inlet 31a penetrating sidewalls of the outer tube 11a and inner tube 17 is formed. The second lumen 18 of the inner tube 17 communicates with outside of the outer tube 11a through the blood inlet 31a. Note that the outer tube 11a and the inner tube 17 are fixed at the whole circumference of the rim of the blood inlet 31a, so that the first lumen 12a does not directly communicate with outside of the outer tube 11a through the blood inlet 31a. Also, the first lumen 12a does not directly communicate with the second lumen 18 through the blood inlet 31a. Note that the both end portions of the second lumen 18 also communicates with the outside of the outer tube 11a. The first lumen 12a communicates with the outside of the outer tube 11a only at a fluid inlet 27a of a connector 25a.

A method for composing the blood inlet 31a is not particularly limited and, for example, a method of fixing the inner tube 17 and outer tube 11a each provided with an opening on its sidewall in advance, so that positions of the opening rims match may be mentioned. Alternately, the blood flow inlet 31a may be formed by fixing the inner tube 17 and the outer tube 11a and, then, forming an opening penetrating side walls of the both at the fixed portion.

A position of forming the blood inlet 31a (that is, an opening at the distal end of the blood flow channel 10a) is on the proximal end side of a boundary 50 with the extraction/contraction portion 3a on the proximal end portion 5a of the balloon 2a. The position is not particularly limited as far as the blood inlet 31a positions in the vessel when the balloon 2a is placed in the aorta. Specifically, as to the position of the blood inlet 31a, it is preferable that the opening rim on the distal end side of the inlet 31a positions on the proximal end side of the boundary 50 with the extraction/contraction portion 3a of the balloon 2a by leaving a distance of (L=) 3 to 300 mm, and it is more preferable to position on the proximal end side by leaving a distance of (L=) 8 to 100 mm.

When providing the blood inlet 31a at the position as above, the blood inlet 31a positions in vessel near the heart, such as an upper portion of the descending aorta, inside the aortic arch portion and inside the left subclavian artery, when placing the balloon 2a in the descending aorta. Therefore, changes of blood pressure caused by the beating of the heart can be measured highly accurately with good response.

Note that the boundary 50 with the extraction/contraction portion 3a on the proximal end portion 5a is, as shown in FIG. 1, a boundary between the proximal end side taper portion 7a of the extraction/contraction portion 3a and the proximal end portion 5a and is a part to be a starting point of the connection margin on the proximal end portion 5a with the outer tube 11a and the inner tube 17.

In the present embodiment shown in FIG. 1, the blood inlet 31a is formed on the proximal end side of the proximal end portion 5a of the balloon 2a, but it may be formed on the proximal end portion 5a. In that case, the blood inlet 31a is formed at a position, where the inner tube 17, the outer tube 11a and the proximal end portion 5a of the balloon 2a are fixed to be one body, by penetrating their sidewalls.

An opening shape of the blood inlet 31a is not particularly limited, but a circular shape or an oval shape having a long axis along the center axis of the outer tube 11a are preferable. Also, an opening area of the blood inlet 31a is preferably 0.2 to 3 mm². When the opening area of the blood inlet 31a is smaller than the 0.2 mm², taking of blood becomes insufficient at the blood inlet 31a and sufficient measurement of the blood pressure changes becomes difficult. While, when forming a blood inlet 31a having an opening area of exceeding 3 mm², it is liable that strength of the catheter tube 8a at the position becomes insufficient.

In terms of keeping airtightness of the first lumen 12a of the outer tube 11a used as the fluid flow channel 9a for expanding the balloon, the inner surface of the outer tube 11a and the outer surface of the inner tube 17 are preferably flat at least around the blood inlet 31a.

It is sufficient if the inner surface of the outer tube 11a and the outer surface of the inner tube 17 are fixed at least around the blood inlet 31a, but it is preferable to be fixed over at least 60% of the entire length of the outer tube, and it is more preferable to be fixed over the entire length of the outer tube 11a. When fixing the inner surface of the outer tube 11a and the outer surface of the inner tube 17 over at least 60% of the entire length of the outer tube 11a, the flow channel resistance against a fluid for expanding the balloon becomes low in the first lumen 12a, so that expansion and contraction of the balloon 2a can be performed without any delay.

The connector 25a composing the catheter tube 8a of the intra-aortic balloon catheter 1a is a molded item provided with a hollow portion having three openings. Namely, the connector 25a has an outer connection opening 26a to be a connection opening of the inner tube 17 and the outer tube 11a, a fluid inlet 27a to be a proximal end opening of the fluid flow channel 9a for balloon expansion, and a blood pressure measurement opening 28a to be a proximal end opening of the blood flow channel 10a for blood measurement.

A length of the connector 25a is normally 10 to 150 mm. As a material composing the connector 25a, ABS (an acrylonitrile butadiene styrene copolymer), polystyrene, polypropyrene, polycarbonate and other thermoplastic resins are preferably used.

In the intra-aortic balloon catheter 1a shown in FIG. 1, the outer tube 11a is connected as a result that the proximal end portion is inserted to the outer tube connection opening 26a of the connector 25a, and the inner tube 17 is connected as a result that the proximal end portion is inserted from the outer tube connection opening 26a to reach to the blood pressure measurement opening 28a.

When using the intra-aortic balloon catheter 1a, the fluid inlet 27a is connected to a pump device for flowing a fluid, such as a helium gas, to and from the balloon 2a, for example, through a tube. The blood pressure measurement opening 28a is connected to a blood pressure measurement apparatus for measuring the blood pressure at the blood inlet 31a (a distal end opening of the blood flow channel 10a), for example through a tube filled with a normal saline solution.

Also, preferably, the outer tube connection opening 26a communicates with the fluid inlet 27a of the connector 25 by a linear channel as shown in FIG. 1. When the outer tube connection opening 26a communicates with the fluid inlet 27a of the connector 25 by a linear channel, flow channel resistance against the fluid for expanding the balloon becomes low in the connector 25a, so that expansion and contraction of the balloon 2a can be performed without any delay.

In the intra-aortic balloon catheter 1a shown in FIG. 1, the inner surface of the proximal end portion 5a of the balloon 2a is joined with the outer surface of the distal end portion of the outer tube 11a, and the distal end portion 4a of the balloon 2a is joined with the distal end portion of the inner tube 17 via the tip 32a. As a result, the balloon 2a is attached to the outer tube 11a and the inner tube 17 (in other words, the distal end portion of the catheter tube 8a). As the joining method, heat sealing and adhesive joining, etc. may be mentioned. Due to the joining, inside of the balloon 2a is brought to be in an airtight state except for the distal end opening of the outer tube 11a.

The tip 32a jointed to the distal end portion of the balloon 2a is a member composed of a relatively soft material and has a function of preventing the distal end portion of the inner tube 17 from perforating the arterial vessel wall. As the material composing the tip 32a, synthetic resin, such as a soft polyvinyl chloride resin, silicone resin, urethane based elastomer, styrene based elastomer, vinyl chloride based elastomer, olefin based elastomer, polyester based elastomer and polyamide based elastomer; or natural rubber, etc. may be used. In terms of the antithronbogenicity, urethane based elastomer is preferably used.

Also, as hardness of the material composing the tip 32a, the Shore A hardness of 50 to 95 is preferable and the Shore A hardness of 65 to 80 is more preferable. When the Shore A hardness is 95 or higher, it is too hard and causes a possibility of perforating the vessel wall, while when the Shore A hardness is 50 or lower, it is too soft and causes a possibility that the distal end portion of the inner tube 17 staves in the tip 32a to perforate the vessel wall. Note that the Shore hardness here indicates a solid state value measured based on the JIS standard K-7215.

Also, by compounding an X-ray contract agent in the material composing the tip 32a, the tip 32a can be observed by X-ray fluoroscopy from the outside of the body when inserting the intra-aortic balloon catheter 1a to the vessel. As the X-ray contract agent, metals, such as gold, platinum, tungsten and lead, or metal compounds, such as a titanium oxide, barium sulfite, bismuth trioxide and bismuth subcarbonate may be mentioned.

A shape of the tip 32a is preferably a tubular shape for a guide wire to penetrate therein. Also, the distal end of the tip 32a is preferably a hemisphere shape. A length of the tip 32a in the axial direction is preferably 5 to 25 mm, and the outer diameter is 1.6 to 3.4 mm, and the inner diameter is preferably 0.1 to 1.5 mm.

### Second Embodiment

As shown in FIG. 2, the intra-aortic balloon catheter 1b of the second embodiment is the same as the intra-aortic balloon catheter 1a of the first embodiment except for the different points explained below. Namely, in the embodiment shown in FIG. 2, instead of the inner tube 17 shown in FIG. 1, a proximal side inner tube 19 and a distal side tube 21 are used, and a recess 13 extending along the axis direction is formed on a part of the outer surface of the outer tube 11b.

As shown in FIG. 2, a catheter tube 8b of the intra-aortic balloon catheter 1b of the second embodiment comprises an outer tube 11b, the proximal side inner tube 19, the distal side inner tube 21 and a connector 25b. The outer tube 11b composing the catheter tube 8b has a tubular shape, and a first lumen 12b is formed therein along the axial direction. The first lumen 12b is used as the fluid flow channel 9b for expanding the balloon of the catheter tube 8b. A size and material of the outer tube 11b may be the same as those of the outer tube 11a of the intra-aortic balloon catheter 1a.

The outer surface of the outer tube 11b is provided with a recess 13 extending in the axial direction. A length of the recess 13 in the axial direction is normally 1 to 12 mm, a width of the recess is normally 0.1 to 3 mm. A depth of the recess 13 is preferably the same as the outer diameter of the proximal side inner tube 19 and is normally 0.5 to 2.1 mm. A method of forming the recess 13 on the outer surface of the outer tube 11b is not particularly limited and it can be formed, for example, by pressing a heated pallet against the outer wall of the outer tube 11b.

The proximal side inner tube 19 composing the catheter tube 8b of the intra-aortic balloon catheter 1b has a tubular shape, wherein a second lumen 20 is formed along the axial direction. The second lumen 20 is used as a blood flow channel 10b for measuring the blood pressure of the catheter tube 8b and also used for leading the guide wire. An inner diameter of the proximal side inner tube 19 is preferably 0.4 to 1.5 mm, and a thickness of the proximal side inner tube is preferably 0.05 to 0.3 mm. A length of the proximal side inner tube 19 in the axial direction is normally 100 to 950 mm. As a material composing the proximal side inner tube 19, the same materials of the inner tube 17 of the intra-aortic balloon catheter 1a of the first embodiment can be used.

The proximal side inner tube 19 is provided inside the first lumen 12b of the outer tube 11b by extending in the axial direction. The proximal end portion of the proximal side inner tube 19 protrudes from the proximal end of the outer tube 11b, and the distal end portion of the proximal side inner tube 19 penetrates the sidewall of the outer tube 11b at the proximal end portion of the recess 13 of the outer tube 11b and is in a state of being exposed to the outside. Namely, the distal end opening of the proximal side inner tube 19 positions outside of the first lumen 12b, and the second lumen 20 of the proximal side inner tube 19 communicates with the outside through the distal end opening. Note that, at the position where the distal end portion of the proximal side inner tube 19 penetrates the outer wall of the outer tube 11b, the outer surface of the proximal side inner tube 19 and the outer wall of the outer tube 11b are airtightly joined. Also, the proximal end portion of the proximal side inner tube 19 is connected to the connector 25b in the same way as the inner tube 17 in the intra-aortic balloon catheter 1a of the first embodiment.

In the intra-aortic balloon catheter 1b of the second embodiment, the entire second lumen 20 of the proximal side inner tube 19 composes the entire blood flow channel 10b for measuring the blood pressure, the distal end opening of the proximal side inner tube 19 is the blood inlet 31b (the distal end opening of the blood flow channel 10b). The position of forming the blood inlet 31b is the same as that in the intra-aortic balloon catheter 1a of the first embodiment.

The distal side inner tube 21 composing the catheter tube 8b of the intra-aortic balloon catheter 1b has a tubular shape, and a third lumen 22 is formed therein along the axial direction. The third lumen 22 leads a guide wire used at the time of inserting the intra-aortic balloon catheter 1b to the patient's artery.

An inner diameter of the distal side inner tube 21 is preferably 0.1 to 1.0 mm, and a thickness of the distal side inner tube 21 is not particularly limited as far as it can support the balloon 2b, but it is preferably 0.05 to 0.4 mm. Also, a length of the distal side inner tube 21 in the axial direction is normally 150 to 570 mm. As a material composing the distal side inner tube 21, the same materials as those of the inner tube 17 of the intra-aortic balloon catheter 1a of the first embodiment may be used.

The distal side inner tube 21 is provided inside the first lumen 12b of the outer tube 11b by extending in the axial direction. The distal end portion of the distal side inner tube 21 protrudes from the distal end of the outer tube 11b, and the proximal end portion of the distal side inner tube 21 penetrates the sidewall of the outer tube 11b at the distal end portion of the recess 13 of the outer tube 11b to be in a state of being exposed to the outside. Namely, the proximal end opening of the distal side inner tube 21 positions outside of the first lumen 12b, and the third lumen 22 of the distal side inner tube 21 communicates with the outside through the proximal end opening. Note that, at the position where the proximal end portion of the distal side inner tube 21 penetrates the sidewall of the outer tube 11b, the outer surface of the distal side inner tube 21 and the inner surface of the outer tube 11b are airtightly joined.

In the intra-aortic balloon catheter 1b shown in FIG. 2, the inner surface of the proximal end portion 5b of the balloon 2b is joined with the outer surface of the distal end portion of the outer tube 11b, and the distal end portion 4b of the balloon 2b is joined with the distal end portion of the distal side inner tube 21 via the tip 32b. As a result, the balloon 2b is attached to the distal side inner tube 21 (in other words, the distal end portion of the catheter tube 8b). As a method of joining them, heat sealing and adhesive joining, etc. may be mentioned. Due to the joining, inside of the balloon 2b is brought to be in an airtight state except for the distal end opening of the outer tube 11b.

A distance of the distal end of the proximal side inner tube 19 and the proximal end of the distal side inner tube 21 is preferably 1 to 10 mm. When the distance is shorter than 1 mm, blood pressure changes in the third lumen 22 of the distal side inner tube 21 affects the blood inlet 31b (the distal end opening of the proximal side inner tube 19) and it may be unable to measure accurate blood pressure. When the distance is longer than 10 mm, strength of the catheter tube 8b at the position may become insufficient.

An inner diameters of the proximal side inner tube 19 and that of the distal side inner tube 21 may be different, particularly, the inner diameter of the distal side inner tube 21 is preferably smaller than that of the proximal side inner tube 19 and, it is more preferable that the inner diameter of the distal side inner tube 21 is 50 to 95% of the inner diameter of the proximal side inner tube 19. It is necessary that the inner diameter of the proximal side inner tube 19 has a certain size to transmit the blood pressure changes at the blood inlet 31b to the proximal end opening (a blood pressure measurement opening 28b) of the proximal side inner tube 19. On the other hand, it is sufficient if the inner diameter of the distal side inner tube 21 is made to be able to lead the guide wire, and the distal side inner tube 21 can be made thinner when the inner diameter is made small, consequently, insertion of the intra-aortic balloon catheter 1b to the patient becomes easier.

A thickness of the proximal side inner tube 19 and that of the distal side inner tube 21 may be also different, particularly, the thickness of the proximal side inner tube 19 is preferably thinner than that of the distal side inner tube 21, and it is more preferable that the thickness of the proximal side inner tube 19 is 50 to 95% of the thickness of the distal side inner tube 21. The distal side inner tube 21 is required to have a certain thickness to support the balloon 2b. On the other hand, the proximal side inner tube 19 is not required to be thick since it positions inside the outer tube 11b and the connector 25b, and when the thickness is made thinner, a cross-sectional area of the flow channel of the fluid flow channel 9b becomes wide and expansion/contraction of the balloon 2b can be performed without any delay.

Note that other configuration of the intra-aortic balloon catheter 1b of the second embodiment is the same as that of the first embodiment, and the same effects as those in the first embodiment can be obtained. Also, in FIG. 1 and FIG. 2, same reference numbers are given to the common members and the explanation thereon are omitted.

### Third Embodiment

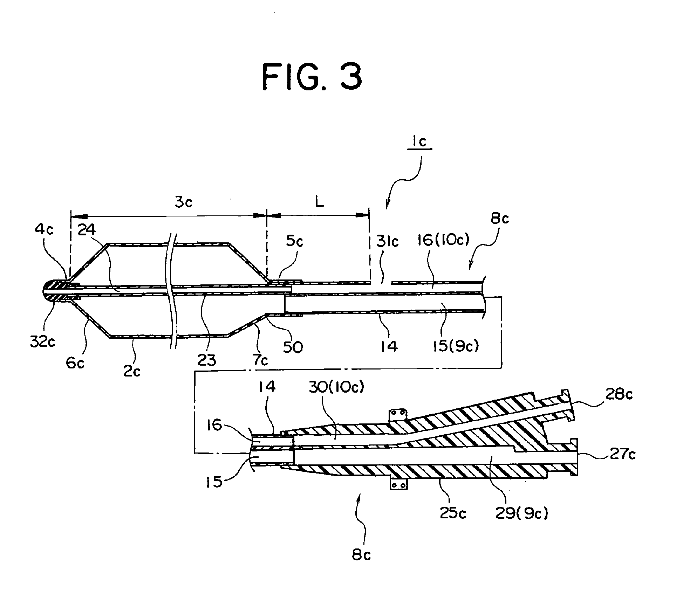

As shown in FIG. 3, a different point of the intra-aortic balloon catheter 1c of a third embodiment from the intra-aortic balloon catheter 1a of the first embodiment is that the catheter tube 8c comprises a two-lumen tube 14, a balloon supporting tube 24 and a connector 25c.

The two-lumen tube 14 composing the catheter tube 8c of the intra-aortic balloon catheter 1c of the third embodiment has a tubular shape, and a first lumen 15 and a second lumen 16 are formed therein in the axial direction. The first lumen 15 is used as a fluid flow channel 9c for balloon expanding of the catheter tube 8c, and the second lumen 16 is used as a blood flow channel 10c for blood pressure measuring of the catheter tube 8c. The second lumen 16 can be used also for leading a guide wire.

An outer diameter of the two-lumen tube 14 is normally 1.2 to 4.0 mm, and a length of the two-lumen tube 14 in the axial direction is normally 400 to 800 mm. Cross-sectional shapes of the first lumen 15 and the second lumen 16 of the two-lumen tube 14 are not particularly limited, but an oval shape is preferable. A cross-sectional area of the first lumen 15 is preferably 0.6 to 6 mm², and a cross-sectional area of the second lumen 16 is preferably 0.01 to 1.7 mm².

As a material for composing the two-lumen tube 14, for example, polyethylene, polypropyrene, polyethylene terephthalate, polyamide, polyvinyl chloride, polyurethane, a fluorine resin or other synthetic resins may be used, and composite materials obtained by reinforcing the synthetic resins by metals may be also used.

A blood inlet 31c is formed on a sidewall of the two-lumen tube 14, and the second lumen 16 communicates with the outside through the blood inlet 31c. A position of forming the blood inlet 31c, that is, a distal end portion of the blood flow channel 10c is the same as that in the intra-aortic balloon catheter 1a of the first embodiment.

Also, an opening shape of the blood inlet 31c is not particularly limited, but a circular shape and an oval shape along the center axis of the two-lumen tube 14 are preferable. Also, an opening area of the blood inlet 31c is preferably 0.2 to 3 mm². When the opening area of the blood inlet 31c is smaller than 0.2 mm², blood cannot be taken in sufficiently at the blood inlet 31c and measurement of the blood pressure changes becomes difficult: While, when a blood inlet 31c having an opening area of exceeding 3 mm² is provided, strength of the catheter tube 8c (two-lumen tube 14) at the position may become insufficient.

A balloon supporting tube 23 composing the catheter tube 8c of the intra-aortic balloon catheter 1c has a tubular shape, and a third lumen 24 is formed therein along the axial direction. A guide wire used at the time of inserting the intra-aortic balloon catheter 1c to the patient's artery is inserted to the third lumen 24. An inner diameter of the balloon supporting tube 23 is preferably 0.1 to 1.0 mm, and a thickness of the balloon supporting tube 23 is not particularly limited as far as it can support the balloon tube 2c, but it is preferably 0.05 to 0.4 mm. A length of the balloon tube 23 in the axial direction is approximately the same as the length of the balloon 2c in the axial direction and is normally 150 to 300 mm. As a material composing the balloon supporting tube 23, the same materials as those of the inner tube 17 of the intra-aortic balloon catheter 1a of the first embodiment can be used.

The proximal end portion of the balloon supporting tube 23 is inserted to the second lumen 16 of the two-lumen tube 14 and joined to be airtight against the outside, so that the third lumen 24 of the balloon supporting tube 23 communicates with the second lumen 16 of the two-lumen tube 14. Also, the inner surface of the proximal end portion 5c of the balloon 2c is joined with the outer surface of the distal end portion of the two-lumen tube 14, and the distal end portion 4c of the balloon 2c is joined with the distal end portion of the balloon supporting tube 23 via the tip 32c. As a result, the balloon 2c is attached to the two-lumen tube 14 and the distal end portion of the balloon supporting tube 23 (in other words, the distal end portion of the catheter tube 8c). As the joining method, heat sealing and adhesive joining, etc. may be mentioned. Due to the joining, inside of the balloon 2c is brought to be in an airtight state except for the distal end opening of the first lumen 15 of the two-lumen tube 14.

The connector 25c composing the catheter tube 8c of the intra-aortic balloon catheter 1c of the third embodiment is a molded item provided with a first duct 29 composing a part of the fluid flow channel 9c for balloon expansion and a second duct 30 composing a part of the blood flow channel 10c for blood pressure measurement being separate to each other.

The connector 25c and the two-lumen tube 14 are connected, so that the first lumen 15 communicates with the first duct 29 and the second lumen 16 communicates with the second duct 30. As a result of the connection, a fluid inlet 27c as a proximal end opening of the first duct 29 of the connector 25c can be used as a proximal end opening of the fluid flow channel 9c for balloon expansion, and a blood measurement opening 28c as a proximal end opening of the second duct 30 can be used as a proximal end opening of the blood flow channel 10c for blood pressure measurement. As a material for composing the connector 25c, the same materials as those of the connector 25a in the intra-aortic balloon catheter 1a of the first embodiment can be used.

Furthermore, the first duct 29 of the connector 25c is preferably configured to be a linear shape. When the first duct 29 is a linear duct, the flow channel resistance against the fluid for balloon expansion becomes low in the first duct 29, so that expansion and contraction of the balloon 2c can be performed without any delay.

Note that other configuration of the intra-aortic balloon catheter 1c of the third embodiment is the same as that in the first embodiment, and the same effects as those in the first embodiment can be obtained.

### How To Use Balloon Catheter

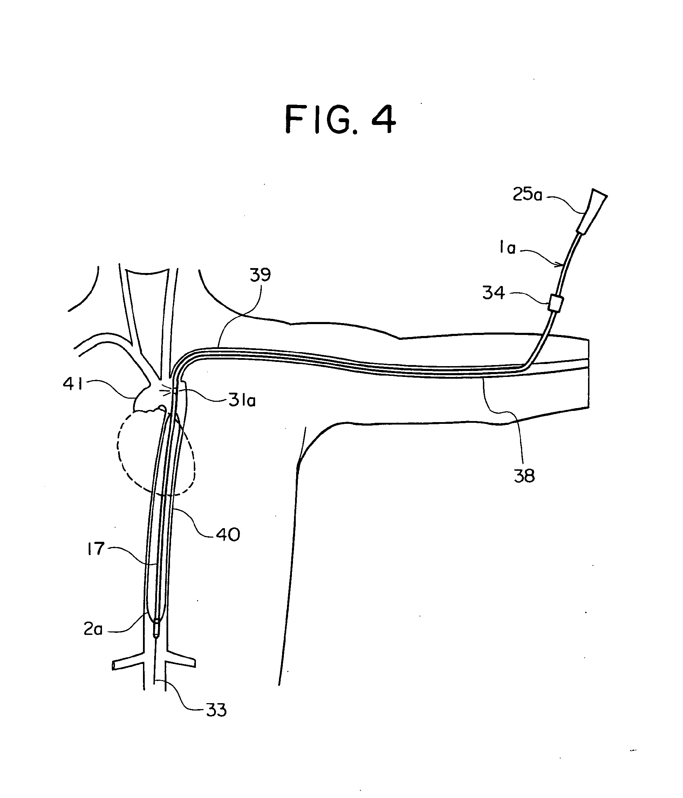

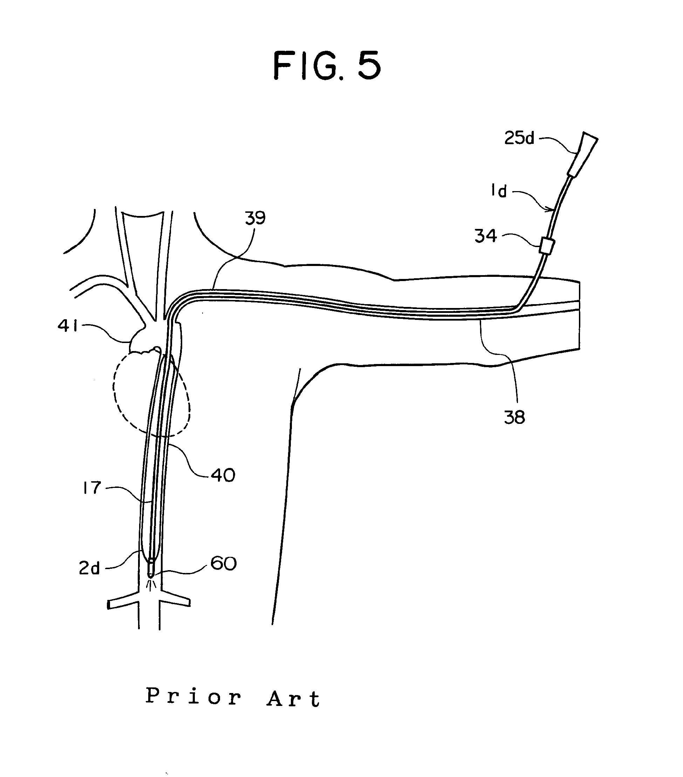

How to use the intra-aortic balloon catheter of the present invention will be explained with reference to the drawings by taking as an example the case of using the intra-aortic balloon catheter 1a of the first embodiment by inserting it from the left brachial artery. FIG. 4 is a schematic view showing a state of using the intra-aortic balloon catheter 1a of the first embodiment according to the intra-aortic balloon catheter of the present invention by inserting it from the left brachial artery, and FIG. 5 is a schematic view showing a state of using an intra-aortic balloon catheter having the conventional configuration by inserting it from the left brachial artery.

First, the balloon 2a of the intra-aortic balloon catheter 1a is wound around the inner tube 17 and a guide wire 33 is inserted to the inner tube 17 (second lumen) in advance. Then, by using a Serdinger method, stick a catheter introducer 34 to the left brachial artery 38 and insert the intra-aortic balloon catheter 1a, in which the guide wire 33 is inserted, to the left brachial artery. Note that it is not always necessary to use the catheter introducer 34, and the guide wire 33 and the intra-aortic balloon catheter 1a may be directly inserted to a perforation provided to the left brachial artery 38.

Next, push the intra-aortic balloon catheter 1a preceded by the guide wire 33 to insert further, so that the balloon 2a passes through the left subclavian artery under the left clavicle 39 and positions in the descending aorta 40. As shown in FIG. 4, when the entire balloon 2a is positioned inside the descending aorta 40, remove the guide wire 33 out, connect a pump device (not shown) to the fluid inlet of the connector 25a, for example, via a tube, and connect a blood pressure measurement device (not shown) to the blood pressure measurement opening, for example, via a tube filled with a normal saline solution. After that, by using the blood pressure measurement device, measure the blood pressure changes transmitted from the blood inlet 31a (distal end opening of the blood flow channel) to the blood pressure measurement opening (proximal end opening of the blood flow channel) of the connector 25a through blood in the blood flow channel, drive the pump device based on the measurement result, and flow a fluid of a helium gas, etc. into or from the balloon 2a through the fluid flow channel. As a result of the operations as above, expansion and contraction of the balloon 2a along with the beating of the heart are performed, and the expansion and contraction of the balloon 2a assist the cardiac function.

As shown in FIG. 5, in the case of the intra-aortic balloon catheter 1d having the conventional configuration,
wherein a distal end opening 60 of the blood flow channel is provided near the distal end of the balloon 2d, the distal end opening 60 of the blood flow channel positions at a lower portion of the descending aorta 40 when inserting it from the arm vessel. Therefore, a blood pressure change caused by the beating of the heart attenuates due to a pressure loss in the descending aorta 40, in which the balloon 2d is inserted, when it reaches to the distal end opening of the blood flow channel, so that the blood pressure change caused by the beating of the heart cannot be measured sufficiently. On the other hand, according to the intra-aortic balloon catheter 1a of the present invention, as shown in FIG. 4, the blood inlet 31a (distal end opening of the blood flow channel) positions on the proximal end side of the boundary 50 (refer to FIG. 1) with the expansion/contraction portion of the balloon 2a. Therefore, when inserting the catheter from the arm vessel, it is possible to position the distal end opening of the blood flow channel close to the heart, such as an upper portion of the descending aorta 40. As a result, attenuation due to pressure loss hardly arises, and blood pressure changes caused by the beating of the heart can be measured highly accurately with good response.

Note that the present invention is not limited to the above embodiments and may be variously modified within the scope of the present invention.

### EXAMPLES

Next, the present invention will be explained based on further detailed examples (animal experiments using a goat).

### Example 1

First, by using an outer tube made by polyamide having a length in the axial direction of 510 mm, an inner diameter of 2.1 mm and a thickness of 0.11 mm, an inner tube made by nickel-titanium alloy having a length in the axial direction of 720 mm, an inner diameter of 0.72 mm and a thickness of 0.11 mm, a balloon made by urethane based elastomer having a length in the axial direction of 200 mm, an inner volume of 30 cc, an outer diameter of 14 mm and a film thickness of 70 pm, a tip made by urethane based elastomer having a length in the axial direction of 10 mm, an inner diameter of 0.72 mm and an outer diameter of 2.1mm and a connector made by ABS; an intra-aortic balloon catheter having the same configuration explained as the first embodiment of the present invention was produced. Note that the blood inlet was formed to have a circular shape having a size of 2.0 mm² on the proximal end side being away by 10 mm from the expansion/contraction portion of the balloon.

Then, a skin of a goat (female, weighing about 43 kg) was incised to expose the carotid artery, and a catheter introducer (an inner diameter was 2.4 mm) was inserted to the carotid artery. Next, the intra-aortic balloon catheter, in which a guide wire (a length in the axial direction of 1500 mm and an outer diameter of 0.5 mm) was inserted, is inserted from the carotid artery of the goat via the catheter introducer. After that, the intra-aortic balloon catheter was pressed to be inserted further, so that the entire balloon was positioned in the descending aorta of the goat. Note that when inserting the intra-aortic balloon catheter from the carotid artery of the goat to position the entire balloon in the descending aorta, in the same way as in the case of inserting from the human's arm artery, the distal end of the balloon positions at a lower portion of the descending aorta and the proximal end of the balloon positions at an upper portion of the descending aorta.

Then, the connector of the intra-aortic balloon catheter was connected to an intra-aortic balloon catheter drive (the product name is IABP Console 907 and made by Zeon Medical Inc.) having a function as a pump device and a blood pressure measurement device. Next, when measuring the blood pressure by operating the blood pressure measurement device in a state of not driving the pump device of the intra-aortic balloon catheter drive, the measured highest blood pressure was 200 mmHg and the lowest blood pressure was 120 mmHg. After that, when performing expansion and contraction of the balloon by a helium gas in a setting of operating the pump device along with one beating in every four beating of the heart by using a blood pressure trigger function (a function of controlling the pump device based on the blood pressure measurement result of the blood pressure measurement device) of the intra-aortic balloon catheter drive, expansion and contraction of the balloon along with the beating of the heart could be performed as the setting.

### Comparative Example 1

First, other than not providing a blood inlet, an intra-aortic balloon catheter of the conventional configuration having the same configuration as that in the intra-aortic balloon catheter used in the example 1 was produced. Then, after taking out the intra-aortic balloon catheter used in the example 1 from the goat, in the same way as in the example 1, the entire balloon of the intra-aortic balloon catheter of the conventional configuration was positioned in the descending aorta of the goat. Next, in the same way as in the example 1, when measuring the blood pressure without performing expansion or contraction, the measured highest blood pressure was 140 mmHg and the lowest blood pressure was 115 mmHg. After that, by using the blood pressure trigger function, in a setting of operating the pump device along with one beating in every four beating of the heart, expansion and contraction of the balloon by a helium gas were attempted, however, because the measured blood pressure changes were not sufficient, the blood pressure trigger function did not work and expansion and contraction of the balloon along with the beating of the heart could not be performed.

### Reference Example 1

First, the intra-aortic balloon catheter having the conventional configuration used in the comparative example 1 was taken out from the goat, the bleeding from the carotid artery was stopped, and the incised skin was sutured. Then, a skin of the goat was incised to expose the femoral artery, and a catheter introducer was inserted to the femoral artery. Next, through the catheter introducer, the entire balloon of the intra-aortic balloon catheter having the conventional configuration was positioned in the descending aorta of the goat.

Note that when inserting the intra-aortic balloon catheter of having the conventional configuration from the femoral artery of the goat and positioning the entire balloon in the descending aorta, in the same way as the case of inserting it from the human's femoral artery, the distal end of the balloon positions at an upper portion of the descending aorta, and the proximal end of the balloon positions at a lower portion of the descending aorta. After that, in the same way as in the example 1, when measuring the blood pressure without performing expansion and contraction of the balloon, the measured highest blood pressure was 190 mmHg and the lowest blood pressure was 110 mmHg. Then, when performing expansion and contraction of the balloon by a helium gas by using the blood pressure trigger function of the intra-aortic balloon catheter drive in a setting of operating the pump device along with one beating in every four beating of the heart, expansion and contraction of the balloon with the beating of the heart as the setting could be performed.

The highest blood pressure and the lowest blood pressure measured in the example 1, comparative example 1 and reference example 1, a difference of the highest blood pressure and the lowest blood pressure, and availability of expansion and contraction of the balloon along with the beating of the heart were listed in Table 1.

**[Table 1]**

| | Highest Blood Pressure (mmHg) | Lowest Blood Pressure (mmHg) | Difference of Highest and Lowest Blood Pressures (mmHg) | Availability of Expansion/ Contraction of Balloon along with Heartbeat |
|---|---|---|---|---|
| Example 1 | 200 | 120 | 80 | Good |
| Comparative Example 1 | 140 | 115 | 25 | No Good |
| Reference Example 1 | 190 | 110 | 80 | Good |

When referring to Table 1, the example 1 and the reference example 1 exhibit approximately the same blood pressure changes, and expansion and contraction of the balloon along with the beating of the heart could be performed. While, in the comparative example 1, a difference of the highest blood pressure and the lowest blood pressure is small and expansion and contraction of the balloon along with the beating of the heart could not be performed. From the results of the animal experiments using the goat, it can be described that when inserting a intra-aortic balloon catheter having the conventional configuration from the human's arm artery, the blood pressure changes cannot be measured highly accurately with good response comparing with the case of inserting it from the femoral artery, and expansion and contraction of the balloon along with the beating of the heart cannot be performed. On the other hand, by using the intra-aortic balloon catheter of the present invention, it can be described that the blood pressure changes can be measured highly accurately with good response, and expansion and contraction of the balloon along with the beating of the heart can be performed even when inserting it from the arm artery.

As explained above, according to the intra-aortic balloon catheter of the present invention, as a result of positioning the blood inlet used for measuring the blood pressure on the proximal end side of the boundary with the expansion/contraction portion on the proximal end portion of the balloon, the blood pressure changes can be measured highly accurately with good response even when inserting it from the arm artery, such as the brachial artery.

## Claims

1. An intra-aortic balloon catheter comprising
a catheter tube having a fluid flow channel for balloon expansion and a blood flow channel for blood pressure measurement, and
a balloon attached to a distal end portion of said catheter tube and having an expansion/contraction portion to be expanded and contracted as a result of flowing a fluid in and out through said fluid flow channel;
wherein
said balloon is joined with said catheter tube at a distal end portion and proximal end portion of said balloon,
a blood inlet is formed on said catheter tube, so that inside of said blood flow channel communicates with outside of said catheter tube, and
said blood inlet positions on the proximal end side of a boundary with said expansion/contraction portion on the proximal end portion of said balloon.

2. The intra-aortic balloon catheter as set forth in claim 1, wherein said blood inlet positions on the proximal end side by leaving a distance of 3 to 300 mm from the boundary with said expansion/contraction portion on the proximal end portion of said balloon.

3. The intra-aortic balloon catheter as set forth in claim 1, wherein an opening area of said blood inlet is 0.2 to 3 mm².

4. The intra-aortic balloon catheter as set forth in claim 1, wherein
said catheter tube comprises an outer tube and an inner tube, wherein at least a part of an outer surface of the inner tube is joined with an inner surface of said outer tube along with the axial direction, so that said fluid flow channel is formed inside of said outer tube, and said blood flow channel is formed in the inner tube;
a distal end of said inner tube protrudes to the distal end side from a distal end of said outer tube;
a distal end portion of said balloon is joined with the distal end portion of said inner tube, and a proximal end portion of said balloon is joined with the distal end portion of said outer tube; and
said blood inlet is formed at a joined portion of said inner tube and outer tube, and said outer tube and inner tube are joined over all circumference of an opening rim of said blood inlet.

5. The intra-aortic balloon catheter as set forth in claim 1, wherein
said catheter tube comprises an outer tube and an inner tube arranged inside of said outer tube along with the axial direction, so that said fluid flow channel is formed inside of said outer tube, and said blood flow channel is formed inside of the inner tube;
a distal end of said inner tube protrudes to the distal end side than a distal end of said outer tube;
a distal end portion of said balloon is joined with a distal end portion of said inner tube, and a proximal end portion of said balloon is joined with a distal end portion of said outer tube;
a recess is formed on a part of the outer surface of said outer tube;
said inner tube is exposed to the outside of said outer tube at said recess; and
said blood inlet is formed on said inner tube positioning inside of said recess.

6. The intra-aortic balloon catheter as set forth in claim 5, wherein:
said inner tube comprises a proximal side inner tube and a distal side inner tube separated from the proximal side tube;
a distal end side opening of said proximal side inner tube positions inside of said recess, and a proximal end side opening of said distal side inner tube positions inside of said recess;
the proximal end side opening and the distal end side opening face to each other by leaving a predetermined distance inside of said recess; and
the distal end side opening of said proximal side inner tube composes said blood inlet.

7. The intra-aortic balloon catheter as set forth in claim 1, wherein:
said catheter tube comprises a two-lumen tube and a balloon supporting tube;
a first lumen composing said fluid flow channel and a second lumen composing said blood flow channel are formed in said two-lumen tube along with the axial direction;
a distal end portion of said two-lumen tube is joined with said balloon supporting tube;
a distal end portion of said balloon supporting tube is joined with a distal end portion of said balloon, and a distal end portion of said two-lumen tube is joined with a proximal end portion of said balloon; and
said blood inlet is formed on a sidewall of said two-lumen tube.

8. The intra-aortic balloon catheter as set forth in claim 7, wherein a third lumen is formed inside of said balloon supporting tube along with the axial direction, and the third lumen communicates with said second lumen.

9. The intra-aortic balloon catheter as set forth in claim 1, configured that, when said balloon is positioned in an aorta, said blood inlet positions in a blood vessel and a proximal end opening of said blood flow channel positions outside of the body.

10. The intra-aortic balloon catheter as set forth in claim 1 used by being inserted from the arm artery.
